# EUROPEAN PATENT APPLICATION

(11) **EP 1 898 216 A2**
(43) Date of publication of application: **12.03.2008**
(21) Application number: 07016631.9
(22) Date of filing: 24.08.2007
(51) Int. Cl.: G01N 33/569

(54) **Diagnostic reagent and diagnostic reagent kit for HIV infection, method for manufacturing diagnostic reagent for HIV infection and method for detecting anti-HIV antibody**

(30) Priority: 28.08.2006 JP 2006231247
(71) Applicant: SYSMEX CORPORATION, Kobe-shi, Hyogo 651-0073 (JP)
(72) Inventor: Yamada, Miho, Kobe-shi Hyogo 651-0073 (JP); Miyaji, Miki, Kobe-shi Hyogo 651-0073 (JP)
(74) Representative: HOFFMANN EITLE

(57) **Abstract**

A diagnostic reagent for HIV infection is described. The diagnostic reagent comprises a first carrier particle for specifically detecting a first anti-HIV antibody and a second carrier particle for specifically detecting a second anti-HIV antibody different from the first anti-HIV antibody. On the first carrier particle, a first HIV antigen for detecting the first anti-HIV antibody is immobilized. On the second carrier particle, a second HIV antigen for detecting the second anti-HIV antibody is immobilized. The second HIV antigen is different from the first HIV antigen.

## Description

### BACKGROUND OF THE INVENTION

### 1. Field of the Invention

The present invention relates to a diagnostic reagent and diagnostic reagent kit for HIV infection. Also, the present invention relates to a method for manufacturing the diagnostic reagent and a method for detecting an anti-HIV antibody.

### 2. Description of the Related Art

Human Immunodeficiency Virus (HIV) is a single-stranded RNA (+ chain) virus having an envelope (env) . HIV belongs to a retrovirus family. There are Human Immunodeficiency Virus type 1 (HIV-1) and Human Immunodeficiency Virus type 2 (HIV-2) in HIV. When HIV has entered a living body, a plurality of kinds of antibodies which can bind to HIV are produced in order to exclude HIV. By obtaining a sample such as blood from the living body, and detecting an anti-HIV antibody in the sample, whether the living body is infected with HIV or not can be tested.

As a method of detecting an anti-HIV antibody in a sample, for example, the method described in Japanese Laid-Open Patent Application Publication No. H7-301633 is known. According to the method, the anti-HIV antibody is detected by using a reagent containing human erythrocyte sensitized with an HIV-1 gag p24 recombinant antigen, and CKS (CTP: CMP-3-deoxy-mannooctylosonate cytidyl transferase or CMP-KDO synthetase).

### SUMMARY OF THE INVENTION

The scope of the present invention is defined solely by the appended claims, and is not affected to any degree by the statements within this summary.

An object of the present invention is to provide a reagent which can detect an anti-HIV antibody in a sample at a higher sensitivity than the previous sensitivity. Another object of the present invention is to provide a method for manufacturing the reagent. A further object of the present invention is to provide a method which can detect an anti-HIV antibody at a higher sensitivity than the previous sensitivity.

A first aspect of the present invention relates to a diagnostic reagent for HIV infection, comprising: a first carrier particle for specifically detecting a first anti-HIV antibody, the first carrier particle on which a first HIV antigen for detecting the first anti-HIV antibody is immobilized; and a second carrier particle for specifically detecting a second anti-HIV antibody different from the first anti-HIV antibody, the second carrier particle on which a second HIV antigen for detecting the second anti-HIV antibody is immobilized, and the second HIV antigen different from the first HIV antigen.

A second aspect of the present invention relates to a diagnostic reagent kit for HIV infection, comprising: the carrier particle reagent; and a labeled antibody reagent comprising a labeled antibody which is capable of binding to the first anti-HIV antibody and the second anti-HIV antibody.

A third aspect of the present invention relates to a method for manufacturing diagnostic reagent for HIV infection, comprising steps of: first immobilizing a first HIV antigen, which is capable of binding to a first anti-HIV antibody, on a first carrier particle; second immobilizing a second HIV antigen, which is different from the first HIV antigen and is capable of binding to a second anti-HIV antibody, on a second carrier particle; and mixing the first carrier particle and the second carrier particle; wherein an HIV antigen other than the first antigen is not substantially immobilized on the first carrier particle, and an HIV antigen other than the second antigen is not substantially immobilized on the second carrier particle.

A forth aspect of the present invention relates to a method for detecting antibody to HIV in sample, comprising steps of: mixing the sample with the carrier particle reagent; and detecting a first complex formed on the first carrier particle, the first complex comprising the first anti-HIV antibody and the first HIV antigen, and a second complex formed on the second carrier particle, the second complex comprising the second anti-HIV antibody and the second HIV antigen.

### BRIEF DESCRIPTION OF THE DRAWINGS

Fig. 1 is a graph showing a counted value of a blank sample, and a counted value of a negative sample.
Fig.2 is a graph showing a counted value of a positive sample.

### DESCRIPTION OF THE PREFERRED EMBODIMENTS

The reagent of one embodiment of the present invention is used for detecting an anti-HIV antibody in a sample. The reagent contains a first carrier particle and a second carrier particle. The first carrier particle is for specifically detecting a first antibody to HIV in a sample. A first antigen which can bind to the first antibody is immobilized on the first carrier particle. An HIV antigen other than the first antigen is not substantially immobilized on the first carrier particle. The second carrier particle is for specifically detecting a second antibody to HIV in a sample. A second antigen which can bind to the second antibody is immobilized on the second carrier particle. An HIV antigen other than the second antigen is not substantially immobilized on the second carrier particle. That is, the first carrier particle is for detecting the first antibody, and the second carrier particle is for detecting the second antibody. By using the reagent comprising the first carrier particle and the second carrier particle, the anti-HIV antibody in a sample can be detected at a high sensitivity. Thereby, it becomes possible to more accurately diagnose the presence or the absence of infection of a living body with HIV. It is preferable that the first carrier particle and the second carrier particle are contained in a buffer solution in a vessel.

A method of immobilizing an antigen on the carrier particle is not particularly limited. As a method of immobilization, an antigen may be directly immobilized on the carrier particle, or may be indirectly immobilized thereon. The indirect immobilization means that an antigen is immobilized on the carrier particle by intervening a particular substance (hereinafter, referred to as an intervening substance) between the carrier particle and an antibody. It is preferable that the intervening substance is two kinds of substances which can bind each other (first intervening substance and a second intervening substance), and are not substantially bind to the anti-HIV antibody. It is preferable to use a hapten as the first intervening substance, and use a substance which binds to this hapten (hereinafter, referred to as a hapten-binding substance) as the second intervening substance. As the hapten, biotin, 2,4-dinitrophenol, 2,4,6-trinitrophenol, and fluorescein isothiocyanate (FITC) can be used. It is preferable that the hapten-binding substance is a substance which can bind two or more hapten molecules simultaneously. For example, an antibody to the hapten can be used as the hapten-binding substance. When biotin is used as the hapten, avidin and streptavidin may be used as the hapten-binding substance.

As an example of the first intervening substance and the second intervening substance, the case of use of a combination of avidin and biotin will be explained. First, avidin (or biotin) is immobilized on a carrier particle, and biotin (or avidin) is bound to an antigen. Then, by binding of the avidin (or biotin) immobilized on the carrier particle and the biotin (or avidin) bound to the antigen, the antigen can be bound to the carrier particle. As the antigen with the hapten such as biotin bound thereto, and the carrier particle on which a hapten-binding substance such as avidin is immobilized, for example, commercially available ones can be used.

The antigen with the hapten bound thereto may be prepared by binding the hapten and an antigen using a cross-linker. The cross-linker is not particularly limited as far as it is a cross-linker which is usually used for binding the hapten and a polypeptide. Specifically, the hapten and a protein can be bound by introducing a functional group into the hapten using the cross-linker, and reacting the introduced functional group and a functional group possessed by an antigen. Alternatively, another functional group may be further introduced into the functional group possesses by the antigen using the cross-linker. For example, a sulfhydryl group is introduced into a terminal amino group of the antigen using the cross-linker. A functional group having high reactivity with the sulfhydryl group (e.g. maleimide group) is introduced into the hapten using the cross-linker. Then, by reacting the sulfhydryl group introduced into the antigen and the functional group introduced into the hapten, the hapten and the antigen can be bound, being not limiting.

A compound which can be used as the cross-linker is not particularly limited. Examples of the cross-linker include 1-ethyl-3-(3-dimethylaminopropyl)carbodiimide (EDAC), N-hydroxysuccinimide (NHS), 2-imonothiolane, N,N'-o-phenylenedimaleimide, N-succinimidyl S-acetylthioacetate (SATA), N-succimimidyl S-acetylpropionate (SATP), N-succinimidyl 3-(2-pyridyldithio)propionate, N-succinimidyl 4-(N-maleimidomethyl) cyclohexane-1-carboxylate, N-succinimidyl 6-maleimidohexanoate, N-succinimidyl 4-iodoacetylaminobenzoate, N-succinimidyl 3-(p-hydroxyphenyl)propionate, N-succinimidyl m-maleimidobenzoate, N-succinimidyl 4-maleimidobutyrate, and N-succinimidyl 4-(p-maleimidophenyl)butyrate. As the cross-linker, one kind of them, or two or more kinds of them can be used.

The first HIV antigen is different from the second HIV antigen. There are HIV-1 and HIV-2 in HIV. Therefore, in order to avoid false-negative, it is preferable that one of the first antigen and the second antigen is an antigen to an anti-HIV-1 antibody, and the other is an antigen to an anti-HIV-2 antibody. It is preferable that the antigen is an antigen of an env region or a gag region of HIV. One of the first antigen and the second antigen may be an antigen of an env region of HIV-1, and the other may be an antigen of a gag region of HIV-1. One of the first antigen and the second antigen may be an antigen of an env region of HIV-2, and the other may be an antigen of a gag region of HIV-2. Examples of the antigen contains p24 antigen of the gag region of HIV-1, gp41 antigen of the env region of HIV-1, gp36 antigen of the env region of HIV-1. It is preferable that the first antigen is the gp41 antigen of HIV-1, and the second antigen is the env gp36 antigen of HIV-2. In addition, the reagent of the present embodiment may further contain a carrier particle on which an HIV antigen other than the aforementioned HIV antigens is immobilized.

The first antigen and the second antigen which are prepared by the known method can be used. For example, a DNA encoding the antigen is incorporated into a suitable plasmid. This recombinant plasmid is transformed into a host such as Escherichia coli and yeast. Then, an antigen (recombinant antigen) can be expressed from the DNA incorporated into the plasmid. Alternatively, an antigen (peptide antigen) can be chemically synthesized using an automatic peptide synthesizer (e.g. Applied Biosystems Model 430A etc.). The first antigen and the second antigen may be either of the recombinant antigen or the peptide antigen.

On the first carrier particle, any of the recombinant antigen and the peptide antigen is immobilized as the first antigen. On the second carrier particle, any of the recombinant antigen and the peptide antigen is immobilized as the second antigen.

In addition to the first carrier particle and the second carrier particle, a third carrier particle may be contained in the reagent. On the third carrier particle, for example, an antigen of the same kind as that of the first antigen, which is prepared by a different method from the method for the first antigen, is immobilized.

For example, the case where a gp41 recombinant antigen of HIV-1 is immobilized on the first carrier particle and a gp36 peptide antigen of HIV-2 is immobilized on the second carrier particle will be explained. In this case, a carrier particle on which a gp41 peptide antigen of HIV-1 is immobilized or a carrier particle on which a gp36 recombinant antigen of HIV-2 is immobilized may be contained in a reagent as the third carrier particle. An anti-HIV-1 antibody which is difficult to react with a recombinant antigen is rarely contained in a sample. For this reason, as described above, not only the first carrier particle, but also a carrier particle on which a gp41 peptide antigen of HIV-1 is immobilized are contained in the reagent, thereby, false-negative is avoided, and an anti-HIV antibody can be detected with better precision.

The carrier particle is not particularly limited, as far as an antigen can be immobilized thereon. Examples of the carrier particle include: cellulose esters such as nitrocellulose and carboxylcellulose; natural or synthetic resins such as latex, rubber, polyethylene, polypropylene, polystyrene, styrene-butadiene copolymer, polyvinyl chloride, polyvinyl acetate, polyacrylamide, polylmethacrylate, styrene-methacrylate copolymer, polyglycidyl methacrylate, acrolein-ethylene glycol dimethacrylate copolymer, and a derivative thereof; particles consisting of inorganic materials such as glass (e.g. activated glass), silica gel, kaolin, talc, silica-alumina, alumina, barium sulfate, cobalt, and iron; and metal colloids such as gold.

It is preferable that the carrier particle is a particle having magnetism (magnetic particle). When the magnetic particle is dispersed in a liquid in a reaction vessel, magnetic particle can be rapidly and simply collected by making a magnet approach from the outside of the vessel. Thereby, discharge of a liquid component in the reaction vessel, and addition of a new other liquid to the reaction vessel can be rapidly and simply performed. Therefore, B/F separation and change of a buffer can be rapidly and easily performed.

It is preferable that, as the carrier particle, a carrier particle having a small particle diameter is used. As the carrier particle has a smaller particle diameter, a surface area per unit mass of the carrier particle is increased. Thereby, a larger amount of an antigen can be immobilized on the carrier particle. A particle diameter of the carrier particle is preferably 0.5 to 5 µm, more preferably 0.5 to 3 µm. A concentration of the carrier particle in the reagent is preferably 0.5 to 20 mg/mL (0.05 to 2 w/v%), more preferably 2 to 15 mg/mL (0.2 to 1.5 w/v%). In particularly, when the carrier particle is a magnetic particle, the magnetic particle can be rapidly collected by using the magnetic particle in the above concentration range.

When the reagent is mixed with a sample containing anti-HIV antibodies, a complex of the first antigen and the first anti-HIV antibody is formed on the first carrier particle, and a complex of the second antigen and the second anti-HIV antibody is formed on the second carrier particle. By detecting the presence or the absence of theses complexes, or quantitatively detecting these complexes, whether an anti-HIV antibody is contained in the sample or not can be determined.

A method of detecting the complex formed on the carrier particle is not particularly limited, but it is preferable to use a labeled antibody. The labeled antibody is an antibody to which the labeling substance binds. It is preferable that the labeled antibody specifically binds to an antigen, an antibody or both of them, constituting a complex. In particular, it is preferable that the labeled antibody is a labeled anti-IgG antibody which binds to an anti-HIV antibody, or a labeled anti-IgM antibody which binds to an anti-HIV antibody. Examples of the labeling substance include substances, which emit a detectable signal, and enzymes. Examples of the detectable signal include fluorescence, emission, coloring, and radiation. Examples of the substance which emits the detectable signal include a fluorescent substance, an emitting substance, a coloring substance, and a radioactive isotope. The enzyme is not particularly limited, but alkaline phosphatase (ALP), peroxidase, glucose oxidase, luciferase, and β-galactosidase are suitably used.

When an enzyme-labeled antibody is used, the enzyme-labeled antibody is bound to a complex on the carrier particle. Then, a substrate for the enzyme is added to a complex bound to the enzyme-labeled antibody. In addition, it is preferable that, before addition of the substrate, a liquid component containing an unreacted labeled antibody in a reaction vessel is removed. The substrate causes a chemical change by an enzyme reaction of the aforementioned enzyme. By detecting a change in the optical state of a reaction solution due to the chemical change of the substrate, the complex can be detected. Examples of the change in the optical state of the reaction solution include a change in emission intensity, fluorescence intensity, and absorbance.

For example, when ALP is used as the enzyme, and p-methoxyphenylphosphoric acid, p-nitrophenylphosphoric acid, thymolphthaleinphosphoric acid, a dioxetane analogue, or a salt or an analogue thereof is used as the substrate, a signal (emission or coloring) is emitted from the substrate by an enzyme reaction. By detecting this signal, an abundance of the labeled enzyme, that is, an abundance of the complex on the carrier particle can be measured.

As in the present embodiment, by using a reagent containing a plurality of kinds of carrier particles, on each of which substantially one kind of antigen is immobilized, a plurality of kinds of antibodies can be detected at a higher sensitivity than a reagent (previous reagent) containing a carrier particle on which a plurality of antigens are immobilized. That is, an anti-HIV antibody can be detected at a higher sensitivity by using a reagent containing both of a carrier particle on which substantially only an antigen to an anti-HIV-1 antibody is immobilized, and a carrier particle on which substantially only an antigen to an anti-HIV-2 antibody is immobilized, than using a reagent containing a carrier particle on which an antigen to an anti-HIV-1 antibody and an antigen to an anti-HIV-2 antibody are immobilized. Further, when the reagent of the present embodiment is used, a background noise upon detection is relatively low. Therefore, it can be said that a ratio of a signal and a background noise (S/N ratio) is higher, and a sensitivity is higher in the reagent of the present embodiment as compared with the previous reagent.

The reagent of the present embodiment can be manufactured as follows:

First, the first carrier particle is dispersed in a suitable buffer solution. To the solution, a first antigen solution containing a first antigen which can bind to a first antibody is added. By a procedure such as stirring, the first antigen is immobilized on the first carrier particle. Substantially an HIV antigen other than the first antigen is not contained in the first antigen solution. For this reason, substantially the HIV antigen other than the first antigen is not immobilized on the first carrier particle. Therefore, the first carrier particle on which the first antigen is immobilized becomes a carrier particle which specifically detects the first antibody in a sample.

Then, the second carrier particle is dispersed in a suitable buffer solution. To the solution, a second antigen solution containing a second antigen which can bind to a second antibody is added. By a procedure such as stirring, the second antigen is immobilized on the second carrier particle. Substantially an HIV antigen other than the second antigen is not contained in the second antigen solution. For this reason, substantially the HIV antigen other than the second antigen is not immobilized on the second carrier particle. Therefore, the second carrier particle on which the second antigen is immobilized becomes a carrier particle which specifically detects the second antibody in a sample.

When the antigen is immobilized on the carrier particle by intervening an intervening substance, the aforementioned cross-linker can be used before immobilization of the antigen to bind the intervening substance to the antigen and/or the carrier particle.

The first carrier particle on which the first antigen is immobilized, and the second carrier particle on which the second antigen is immobilized are mixed, and these particles are dispersed in a suitable buffer solution, thereby, the reagent of the present embodiment can be obtained.

Alternatively, a carrier particle on which an HIV antigen of a different kind from the first antigen and the second antigen is immobilized is prepared, and the prepared carrier particle may be contained in the reagent.

A reagent kit which is one embodiment of the present invention comprises an anti-HIV antibody detecting reagent comprising the first carrier particle and the second carrier particle, and a labeled antibody reagent comprising the labeled antibody. Further, when an enzyme-labeled antibody is used as the labeled antibody, it is preferable that a substrate reagent comprising a substrate for the enzyme is contained.

### Example

### 1) Sample

Blood was taken from one HIV non-infected person, and serum prepared from the blood was used as a negative sample. Blood was taken from two HIV carriers, respectively, and each serum prepared from each blood was used as a positive sample 1 and a positive sample 2. In addition, as a blank sample, purified water was prepared.

### 2) Preparation of reagent

### R1 reagent (reaction buffer)

A solution containing 0.1 M triethanolamine (pH 7.6), 0.1 w/v% BSA, and 0.1 w/v% NaN₃ was prepared, which was used as an R1 reagent.

### R2-1 reagent (antigen-immobilized particle reagent)

To a buffer A (20 mM NaPB (pH 7.5)), a commercially available streptavidin-immobilized magnetic particle (average particle diameter 2 µm) was added to a concentration of 0.5 w/v%, and a first recombinant antigen solution (5 µg/mL HIV-1 gp41 recombinant antigen bound to biotin (15 kDa)) was further added thereto. This mixed solution was stirred at room temperature for 1 hour. After stirring, the solution was centrifuged to settle a magnetic particle, and a solution component was discarded. To the resulting magnetic particle, a buffer B (2 w/v% BSA, 0.01 M GTA (pH 8.0), and 50 mM MgCl₂) was added, followed by BSA blocking. This was centrifuged to settle a magnetic particle, and a solution component was discarded. To the resulting magnetic particle, a buffer C (0.1 M GTA buffer (pH 7.7), 2.0 w/v% BSA, and 0.1% NaN₃) was added. This was used as a first antigen-immobilized particle reagent A.

To the buffer A, a commercially available streptavidin-immobilized magnetic particle (average particle diameter 2 µm) was added to a concentration of 0.5 w/v%, and a first peptide antigen solution (1.25 µg/mL HIV-1 gp41 peptide antigen bound to biotin (4 kDa)) was further added thereto. This mixed solution was stirred at room temperature for 1 hour. After stirring, the solution was centrifuged to settle a magnetic particle, and a solution component was discarded. To the resulting magnetic particle, the buffer B was added, followed by BSA blocking. This was centrifuged to settle a magnetic particle, and a solution component was discarded. To the resulting magnetic particle, the buffer C was added. This was used as a first antigen-immobilized particle reagent B.

According to the same manner as that of the first antigen-sensitized particle reagent B except that the second antigen solution (1.25 µg/mL HIV-2 gp36 peptide antigen bound to biotin (4 kDa)) was used in place of the first peptide antigen solution, a second antigen-sensitized particle reagent was prepared.

The first antigen-sensitized particle reagent A, the first antigen-sensitized particle reagent B and the second antigen-sensitized particle reagent were mixed at a ratio of 6:3:1, and this mixed solution was used as an R2-1 reagent. A particle concentration in the mixed solution was 0.5 w/v%.

### R3 reagent (ALP labeled antibody reagent)

A solution containing the following substances was prepared, and this was used as an R3 reagent.
3.5 ng/mL ALP labeled mouse anti-human IgG monoclonal antibody
0.1 M MRS(2-(N-Morpholine) ethanesulfonic acid) pH 6.5
0.15 M NaCl
1 mM MgCl₂
0.1 mM ZnCl₂
0.1 w/v% NaN₃

### R4 reagent (dispersion reagent)

A solution containing the following substances was prepared, and this was used as a R4 reagent.
0.1 M 2-amino-2-methyl-1-propanol (AMP) pH 9.6
1 mM MgCl₂
0.1 w/v% NaN₃

### R5 reagent (substrate reagent)

A solution containing the following substances was prepared, and this was used as an R5 reagent.
CDP-Star with Sapphirine-II (analogue of dioxetane (emitting substrate of ALP), manufactured by Applied Biosystems)

### Washing reagent

A solution containing the following substances was prepared, and this was used as a washing reagent. 20 mM Tris pH 7.4
0.1 w/v% Tween 20
0.1 w/v% NaN₃
0.8 w/v% NaCl

### 3) Measurement

The R1 reagent 120 µL and a sample 10 µL were mixed to react at 42°C for 2.25 minutes. Then, 30 µL of the R2-1 reagent was added to this mixed solution, followed by a reaction at 42°C for 1.5 minutes.

After the reaction, a magnetic particle was settled by centrifugation, and the solution was discarded. To the resulting magnetic particle, the washing reagent was added, the magnetic particle was settled by centrifugation, and the solution was discarded. This was repeated three times, 100 µL of the R3 reagent was added to the magnetic particle, followed by a reaction at 42°C for 2.75 minutes.

After the reaction, the magnetic particle was settled by centrifugation, and the solution was discarded. To the resulting magnetic particle, the washing reagent was added, the magnetic particle was settled by centrifugation, and the solution was discarded. After this was repeated three times, 30 µL of the R4 reagent and 100 µL of the R5 reagent were added to the magnetic particle, followed by a reaction at 42°C for 5 minutes.

Light emission intensity of the reaction solution was measured with a light emission measuring apparatus, and light emission intensity was calculated as a counted value.

### Comparative Example 1

An R2-2 reagent was prepared as follows: In the Comparative Example 1, according to the same manner as that of the above Example except that the R2-2 reagent was used in place of the R2-1 reagent, a counted value of light emission intensity was calculated.

To the buffer A, a streoptavidin-immobilized magnetic particle was added to a concentration of 0.5 w/v%, and a mixed antigen solution (3 µg/mL of HIV-1 gp41 recombinant antigen bound to biotin (15 kDa), 0.375 µg/mL of HIV-1 gp41 peptide antigen bound to biotin (4 kDa), and 0.125 µg/mL of HIV-2 gp36 peptide antigen bound to biotin (4 kDa)) was further added thereto. This mixed solution was stirred at room temperature for 1 hour. After stirring, the solution was centrifuged to settle the magnetic particle, and the solution component was discarded. To the resulting magnetic particle, the buffer C was added. This was used as an R2-2 reagent.

### Comparative Example 2

An R2-3 reagent was prepared as follows: In the Comparative Example 2, according to the same manner as that of the above Example except that the R2-3 reagent was used in place of the R2-1 reagent, a counted value of light emission intensity was calculated.

To the buffer A, a streoptavidin-immobilized magnetic particle was added to a concentration of 0.5 w/v%, and a mixed antigen solution (10 µg/mL of HIV-1 gp41 recombinant antigen bound to biotin (15 kDa), 0.75 µg/mL of HIV-1 gp41 peptide antigen bound to biotin (4 kDa), and 0.25 µg/mL of HIV-1 gp36 peptide antigen bound to biotin (4 kDa)) was further added thereto. This mixed solution was stirred at room temperature for 1 hour. After stirring, the solution was centrifuged to settle the magnetic particle, and the solution component was discarded. To the resulting magnetic particle, the buffer C was added. This was used as an R2-3 reagent.

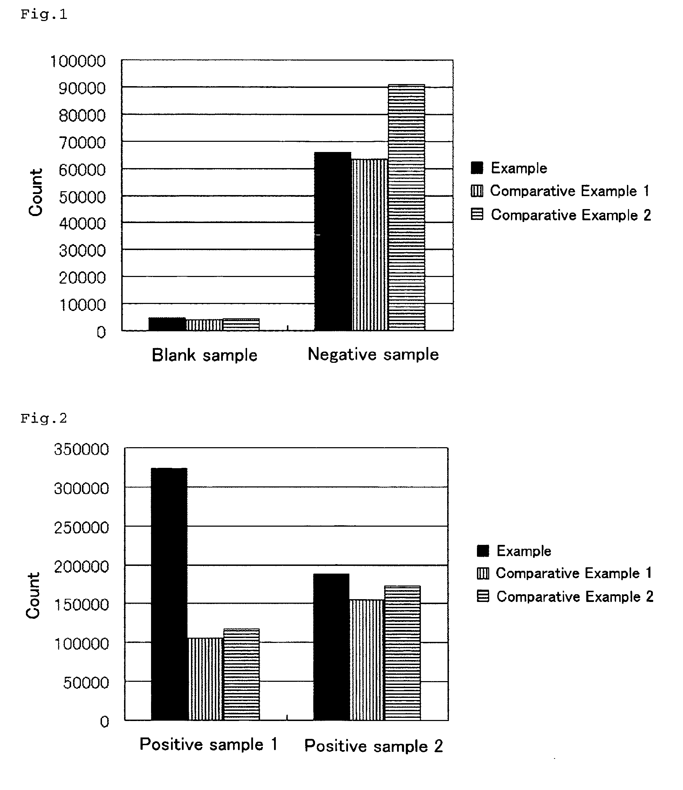

Detection results of Example, Comparative Example 1 and Comparative Example 2 are shown in Table 1, Fig.1 and Fig.2.

**Table 1**

| | Example | Comparative Example 1 | Comparative Example 2 |
|---|---|---|---|
| Blank sample | 4591 | 4087 | 4528 |
| Negative sample | 65822 | 63425 | 90972 |
| Positive sample 1 | 322866 | 106130 | 117738 |
| Positive sample 2 | 188088 | 154536 | 172583 |

Form Table 1 and Fig. 1, in a measurement of a blank sample, little light emission was detected even when any of the R2-1 reagent (Example), the R2-2 reagent (Comparative Example 1) and the R2-3 reagent (Comparative Example 2) was used. In addition, measurement of a negative sample, a lower counted value was obtained using the R2-1 reagent (Example) or the R2-2 reagent (Comparative Example 1) than using the R2-3 reagent.

From Table 1 and Fig.2, in a measurement of any of a positive sample 1 and a positive sample 2, a higher counted value was obtained using the reagent of Example than the reagent of Comparative Example 1 or the reagent of Comparative Example 2. Thereby, it was shown that an HIV antibody could be detected at a higher sensitivity using the reagent of Example than using the reagent of Comparative Example 2.

In addition, when the reagent of Example is used, a lower counted value was obtained in a measurement of a negative sample as described above. Thereby, it was shown that a S/N ratio (ratio of signal (S) and background noise (N)) in Example was high as compared with Comparative Examples 1 and 2. From the foregoing, by using the reagent of the present Example, a pathogen can be detected at a high sensitivity. For this reason, an infectious disease can be diagnosed with better precision using this detection result.

## Claims

1. A diagnostic reagent for Human Immunodeficiency Virus (HIV) infection, comprising:
a first carrier particle for specifically detecting a first anti-HIV antibody, the first carrier particle on which a first HIV antigen for detecting the first anti-HIV antibody is immobilized; and
a second carrier particle for specifically detecting a second anti-HIV antibody different from the first anti-HIV antibody, the second carrier particle on which a second HIV antigen for detecting the second anti-HIV antibody is immobilized, and the second HIV antigen different from the first HIV antigen.

2. The diagnostic reagent according to claim 1, wherein
the first HIV antigen is immobilized on the first carrier particle via a hapten, bound to the first carrier particle, and a hapten-binding substance, which is capable of binding to the hapten, and
the second HIV antigen is immobilized on the second carrier particle via a hapten, bound to the second carrier particle, and a hapten-binding substance, which is capable of binding to the hapten.

3. The diagnostic reagent according to claim 2, wherein the hapten is biotin and the hapten-binding substance is avidin.

4. The diagnostic reagent according to any one of claims 1 to 3, wherein an HIV antigen other than the first HIV antigen is not substantially immobilized on the first carrier particle, and an HIV antigen other than the second HIV antigen is not substantially immobilized on the second carrier particle.

5. The diagnostic reagent according to any one of claims 1 to 4, wherein the first HIV antigen is a Human Immunodeficiency Virus type 1 (HIV-1) antigen, and the second HIV antigen is a Human Immunodeficiency Virus type 2 (HIV-2) antigen.

6. The diagnostic reagent according to claim 5, wherein the first HIV antigen is an HIV-1 recombinant antigen or an HIV-1 peptide antigen, and the second HIV antigen is an HIV-2 peptide antigen.

7. The diagnostic reagent according to claim 6, further comprising a third carrier particle for specifically detecting the first anti-HIV antibody, the third carrier particle on which a third HIV antigen for detecting the first anti-HIV antibody,
wherein the first HIV antigen is the HIV-1 recombinant antigen, and the third HIV antigen is an HIV-1 peptide antigen.

8. The diagnostic reagent according to any one of claims 5 to 7, wherein the first HIV antigen is an antigen of the env region of HIV-1, and the second HIV antigen is an antigen of the env region of HIV-2.

9. The diagnostic reagent according to claim 8, wherein the first HIV antigen is a gp41 antigen of env region of HIV-1, and the second HIV antigen is a gp36 antigen of env region of HIV-2.

10. The diagnostic reagent according to claim 9, further comprising a third carrier particle for specifically detecting the first anti-HIV antibody, the third carrier particle on which a third HIV antigen for detecting the first anti-HIV antibody is immobilized,
wherein the first HIV antigen is a gp41 recombinant antigen of env region of HIV-1, and the third HIV antigen is a gp41 peptide antigen of env region of HIV-1.

11. The diagnostic reagent according to any one of claims 1 to 10, wherein the first carrier particle and the second carrier particle are a magnetic particle.

12. The diagnostic reagent according to any one of claims 1 to 11, further comprising a buffer solution.

13. A diagnostic reagent kit for Human Immunodeficiency Virus (HIV) infection, comprising:
a carrier particle reagent according to claim 1; and
a labeled antibody reagent comprising a labeled antibody which is capable of binding to the first anti-HIV antibody and the second anti-HIV antibody.

14. The diagnostic reagent kit according to claim 13, further comprising a substrate reagent comprising a substrate for an enzyme,
wherein the labeled antibody is an antibody labeled with the enzyme.

15. A method for manufacturing diagnostic reagent for Human Immunodeficiency Virus (HIV) infection, comprising steps of:
first immobilizing a first HIV antigen, which is capable of binding to a first anti-HIV antibody, on a first carrier particle;
second immobilizing a second HIV antigen, which is different from the first HIV antigen and is capable of binding to a second anti-HIV antibody, on a second carrier particle; and
mixing the first carrier particle and the second carrier particle;
wherein
an HIV antigen other than the first antigen is not substantially immobilized on the first carrier particle, and
an HIV antigen other than the second antigen is not substantially immobilized on the second carrier particle.

16. The method according to claim 15, wherein
in the first immobilizing step, the first HIV antigen is immobilized on the first carrier particle via a hapten, bound to the first carrier particle, and a hapten-binding substance, which is capable of binding to the hapten, and
in the second immobilization step, the second HIV antigen is immobilized on the second carrier particle via a hapten, bound to the second carrier particle, and a hapten-binding substance, which is capable of binding to the hapten.

17. The method according to claim 15 or 16, wherein the first HIV antigen is a Human Immunodeficiency Virus type 1 (HIV-1) antigen, and the second HIV antigen is a Human Immunodeficiency Virus type 2 (HIV-2) antigen.

18. A method for detecting antibody to Human Immunodeficiency Virus (HIV) in sample, comprising steps of:
mixing the sample with a carrier particle reagent according to claim 1; and
detecting
a first complex formed on the first carrier particle, the first complex comprising the first anti-HIV antibody and the first HIV antigen, and
a second complex formed on the second carrier particle, the second complex comprising the second anti-HIV antibody and the second HIV antigen.

19. The method according to claim 18, wherein in the detecting step,
a labeled antibody, which is capable of binding to the first antibody and the second antibody, is added to a mixture obtained in the mixing step, and
the first complex and the second complex are detected based on a label of the labeled antibody.

20. The method according to claim19, wherein in the detecting step, an anti-HIV antibody in the sample is detected by quantitating the first complex and the second complex.
